# EUROPEAN PATENT APPLICATION

(11) **EP 4 338 685 A1**
(43) Date of publication of application: **20.03.2024**
(21) Application number: 22806548.8
(22) Date of filing: 29.04.2022
(51) Int. Cl.: A61B 10/02

(54) **ELECTROSURGICAL BIOPSY NEEDLE, ELECTROSURGICAL BIOPSY NEEDLE KIT, AND VACUUM-ASSISTED BREAST BIOPSY SYSTEM**

(30) Priority: 11.05.2021 CN 202110512924; 28.02.2022 CN 202220427922 U
(71) Applicant: Chongqing Xishan Science & Technology Co.,Ltd., Chongqing 401121 (CN)
(72) Inventor: GUO, Yijun, Chongqing 401121 (CN); LI, Mingxuan, Chongqing 401121 (CN)
(74) Representative: Mohun, Stephen John
(86) International application number: PCT/CN2022/090236
(87) International publication number: WO 2022/237580

(57) **Abstract**

An electrosurgical biopsy needle (100), an electrosurgical biopsy needle (100) kit, and a vacuum-assisted breast biopsy system. The electrosurgical biopsy needle (100) comprises: a puncturing component (110), the puncturing component (110) comprising a puncture tube (112), the puncture tube (112) having a front end and a rear end, and a tube wall of the puncture tube (112) being provided with a sampling groove (112a); a cutting component (120), the component (120) comprising a cutting member (122), wherein the cutting member (122) can move back and forth along the axial direction of the puncture tube (112), the cutting component (120) is provided with a first electrode, and the puncturing component (110) is provided with a second electrode; further comprised are a first interface (162) and a second interface (164) that are electrically connected to the first electrode and the second electrode, respectively, either one of the first interface (162) and the second interface (164) being used for direct or indirect connection with a high-frequency output end of a host (300), and the other interface being used for direct or indirect connection with a high-frequency input end of the host (300). The electrosurgical biopsy needle (100) cuts tissue by using high frequency electric waves between a cutting edge (122b) and the puncture tube (112), and has the advantages of high safety, easy operation, a simple structure and low costs.

## Description

### TECHNICAL FIELD

The present disclosure relates to the technical field of medical devices, particularly relates to an electrosurgical biopsy needle, an electrosurgical biopsy needle kit, and a vacuum-assisted breast biopsy system.

### BACKGROUND

Vacuum-assisted breast biopsy system is a medical device for minimally invasive treatment or biopsy of breast mass (tumors). The vacuum-assisted breast biopsy system mainly consists of a host, a biopsy surgical device and a vacuum negative pressure system. Guided by an imaging equipment (such as B-type ultrasound examination), the system punctures a biopsy needle of biopsy surgical device to the surgical location, and then by electrical control, the biopsy needle partially or completely rotary cuts the lesion tissue through mechanical motion, and the rotary cut tissue specimen is ultimately transported to outside through negative pressure, which in turn used for film preparation and pathological analysis and diagnosis.

At present, the rotary cutter head of the biopsy surgical device has weak durability for calcification foci, and may cause bleeding during the process of rotary cutting. The cavity of the rotary cut tissue after operation needs to be pressed for a long time to stop bleeding, and hematoma may appear during recovery. In order to solve this problem, there is an electrosurgical biopsy surgical device in conventional technologies, whose cutting blade of the cutting member is transformed into surgical electrode, high-frequency current passes through the cutting blade of the cutting tube and is conducted to the lesion tissue to be cut, simultaneously cutting tissue and stopping bleeding. However, during the surgery, it is required in this electrosurgical biopsy surgical device that a large-area metal plate is contacted with patient's buttock or thigh as a neutral electrode, the current flows through the human body and then flows back from the neutral electrode to the host to form a circuit. If the neutral electrode and the human skin fit poor contact, it will generate high energy locally, which is prone to scald and the safety is poor. Additionally, the volume of the metal plate is large, and the weight is heavy, which is inconvenient to operate.

### SUMMARY

Accordingly, the present disclosure discloses an electrosurgical biopsy needle, an electrosurgical biopsy needle kit, and a vacuum-assisted breast biopsy system with good safety and simple operation.

In one aspect, an electrosurgical biopsy needle is provided in the present disclosure, including:
a puncture assembly including a puncture tube having a front end and a rear end, a tube wall of the puncture tube is provided with a sampling groove, and a cutting assembly including a cutting member. The cutting member can move back and forth along an axis of the puncture tube. The cutting assembly has a first electrode, and the puncture assembly has a second electrode. The cutting assembly further includes a first interface and a second interface electrically connected to the first electrode and the second electrode, respectively, any one of the first interface and the second interface is configured to be directly or indirectly connected to a high-frequency output terminal of a host, and the other one is configured to be directly or indirectly connected to a high-frequency input terminal of the host.

In one embodiment, the cutting member includes a cutting member body and a cutting blade provided on a front end opening of the cutting member body. The cutting member body is insulated. The cutting blade is electrically conductive and forms the first electrode, and the puncture tube is electrically conductive and forms the second electrode.

In one embodiment, the cutting blade is shaped as a circle or an arc extending along a periphery of the front end opening.

In one embodiment, the cutting blade is formed by bending a metal wire.

In one embodiment, the front end opening is provided with a closed or non-closed metal sheet extending along the periphery of the front end opening, the cutting blade is formed at a front end of the metal sheet, and the metal sheet is electrically connected to the first interface.

In one embodiment, the cutting blade is directly fixed on an end surface of the front end opening.

In one embodiment, the cutting blade is relatively provided on a front side of the front end opening at a certain distance, at least one electrically conductive leg is provided between the cutting blade and the front end opening, one end of the leg is electrically connected to the cutting blade, and the other end thereof is electrically connected to the first interface.

In one embodiment, the cutting blade is shaped as a circle, two legs are provided, and a line connecting the two legs extends through a center of the circle.

In one embodiment, the cutting blade is shaped as an arc, three legs are provided, one end of two of the legs is connected to both ends of the arc, and one end of the remaining one of the legs is connected to an apex of the arc.

In one embodiment, the cutting member body includes a tube body made of metal and an insulation layer wrapping a surface of the tube body, and the tube body is electrically connected to the cutting blade and the first interface.

In one embodiment, the cutting member body is made of an insulation material, at least one connecting wire is provided in a tube wall of the cutting member body, one end of the connecting wire is electrically connected to the cutting blade, and the other end thereof is electrically connected to the first interface.

In one embodiment, the puncture assembly further includes a tubular housing. The rear end of the puncture tube is inserted and fixed in a front end of the housing, and a rear end of the cutting member extends out of the puncture tube and extends into the housing. A pushing rod is fixed and sleeved on the cutting member extending into the housing, and a first gear is threadedly mated to the pushing rod. The puncture assembly further includes a stop-rotation mechanism configured to limit the cutting member from rotating relative to the puncture tube.

In one embodiment, the cutting member includes the cutting member body. An electrode sleeve ring is provided on the cutting member body. The first electrode is connected to the first interface through the electrode sleeve ring. Any one of the first interface and the second interface is further configured to be directly or indirectly connected to a high-frequency output terminal of a host, and the other one is further configured to be directly or indirectly connected to a high-frequency input terminal of the host.

In one embodiment, a rear end of the cutting member body has an electrically conductive area electrically connected to the first electrode. The electrically conductive area extends through a central hole of the electrode sleeve ring and can move back and forth and rotate relative to an axis of the electrode sleeve ring. The electrode sleeve ring is provided with at least one elastic contact assembly. The elastic contact assembly includes an elastic contact provided in the central hole of the electrode sleeve ring. Ahead of the elastic contact is in slidably fit with an outer wall of the electrically conductive area, and the elastic contact is directly or indirectly connected to the first interface.

In one embodiment, the electrode sleeve ring further includes a connector provided on an outer peripheral surface of the electrode sleeve ring. The connector is connected to the first interface, and the elastic contact is electrically connected to the connector.

In one embodiment, the cutting member can rotate relative to the puncture tube.

In one embodiment, the puncture assembly further includes a tubular housing. The rear end of the puncture tube is inserted and fixed in a front end of the housing, and a rear end of the cutting member extends out of the puncture tube and extends into the housing. The first interface and the second interface are provided on the housing.

In one embodiment, the first interface includes a first pin, and the second interface includes a second pin. The first pin is electrically connected to the first electrode. An inner end of the second pin is electrically connected to the puncture tube through a wire, and both outer ends of the first pin and the second pin are exposed from the housing.

In another aspect, an electrosurgical biopsy needle kit is provided in the present disclosure, including a handle. The handle includes a housing and a circuit board provided in the housing. The electrosurgical biopsy needle kit further includes the electrosurgical biopsy needle. The handle further includes a third interface and a fourth interface mated with the first interface and the second interface, respectively. The third interface and the fourth interface are electrically connected to the circuit board. The electrosurgical biopsy needle is connected to the housing. The first interface and the second interface are electrically connected to the third interface and the fourth interface, respectively.

In another aspect, an electrosurgical biopsy needle kit is provided in the present disclosure, including a handle. The handle includes a housing. The electrosurgical biopsy needle kit further includes the electrosurgical biopsy needle, and the electrosurgical biopsy needle kit is electrically connected to the housing.

In another aspect, a vacuum-assisted breast biopsy system is provided in the present disclosure, including: the electrosurgical biopsy needle kit and a host. The host includes a high-frequency transmitting module configured to generate high frequency. The high-frequency transmitting module has a high-frequency output terminal and the high-frequency input terminal. The high-frequency output terminal and the high-frequency input terminal are connected to the circuit board through a cable and thus indirectly connected to the first interface and the second interface, or the high-frequency output terminal and the high-frequency input terminal are directly connected to the first interface and the second interface through the cable.

According to the aforementioned technical solutions, compared with conventional rotary cutting electrosurgical biopsy needles, the electrosurgical biopsy needle, the electrosurgical biopsy needle kit, and the vacuum-assisted breast biopsy system disclosed in the present disclosure can more effectively crush the calcification foci, and can play a traumatic coagulation role due to thermal effect, thus effectively avoiding the risk of intraoperative bleeding and postoperative hematoma. Compared with existing electrosurgical biopsy needles, the puncture assembly of the electrosurgical biopsy needle according to the present disclosure is provided with a first electrode, and the cutting assembly is provided with a second electrode. Cutting is performed using the high-frequency electric wave between the first electrode and the second electrode, such that the range of high-frequency electrical energy is limited to a portion between the two electrodes, and the damage degree and influence range of tissue are much less than single electrode method. In addition, an additional metal plate is not required during operation, which can avoid ambustion of the human body due to poor fitting of the metal plate with human skin. The safety is good and the operation is convenient. Besides, the structure of this electrosurgical biopsy needle is simple and the cost is low.

### BRIEF DESCRIPTION OF THE DRAWINGS

FIG. 1 is a cross-sectional view of an electrosurgical biopsy needle kit according to a first embodiment of the present disclosure.
FIG. 2 is an exploded view of the electrosurgical biopsy needle kit shown in FIG. 1.
FIG. 3 is a perspective view of a puncture assembly of the electrosurgical biopsy needle kit shown in FIG. 1.
FIG. 4 is a perspective view of a cutting assembly of the electrosurgical biopsy needle kit shown in FIG. 1.
FIG. 5 is a partial enlarged view of portion A in FIG. 4.
FIG. 6 is a partial enlarged view of portion C in FIG. 1.
FIG. 7 is a perspective view of a cutting blade with a second structure.
FIG. 8 is a perspective view of a cutting blade with a third structure.
FIG. 9 is a perspective view of a cutting blade with a fourth structure.
FIG. 10 is a partial enlarged view of portion B in FIG. 1.
FIG. 11 is a schematic view of a vacuum-assisted breast biopsy system having the electrosurgical biopsy needle kit shown in FIG. 1.
FIG. 12 is a perspective view of an electrosurgical biopsy needle kit according to a second embodiment of the present disclosure.
FIG. 13 is a schematic view of a vacuum-assisted breast biopsy system having the electrosurgical biopsy needle kit shown in FIG. 12.
FIG. 14 is a perspective view of a cutting assembly of the electrosurgical biopsy needle kit shown in FIG. 1.
FIG. 15 is a perspective view of an electrode sleeve ring of the cutting assembly shown in FIG. 14.

### Description of the reference numerals:

100, electrosurgical biopsy needle; 110, puncture assembly; 112, puncture tube; 112a, sampling groove; 112b, tip portion; 114, housing; 120, cutting assembly; 122, cutting member; 122a, cutting member body; 122b, cutting blade; 122c, vent port; 122d, front end opening; 122e, leg; 122f, metal sheet; 1221, electrically conductive area; 124, pushing rod; 124a, external thread; 124b, limit groove; 130, first gear; 131, threaded hole; 140, shaft sleeve; 142, limiting projection; 150, collection box; 162, first interface; 164, second interface; 170, electrode sleeve ring; 171, mount; 172, elastic contact assembly; 172a, elastic contact; 173, connector; 200, handle; 210, housing; 212, latching groove; 214, motor; 216, second gear; 217, third interface; 218, fourth interface; 300, host; 410, first cable; 420, second cable; 430, third cable; 500, vacuum tube.

### DETAILED DESCRIPTION OF EMBODIMENTS

The present disclosure will be described in detail hereinafter with reference to the accompany drawings and in combinations with the embodiments. It should be noted that the following embodiments and features in the embodiments can be combined with each other under the premise of no conflict.

The orientation terms herein, such as front, back, top, bottom, and the like involved are defined by locations of the parts in the accompanying drawings and locations of the parts among each other, only for clarity and convenience of expression of the technical solution. It should be understood that the use of the orientation terms shall not limit the scope of protection claimed in the present disclosure.

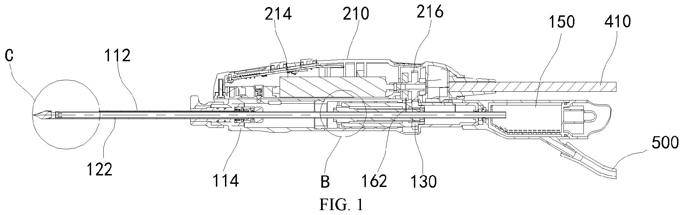

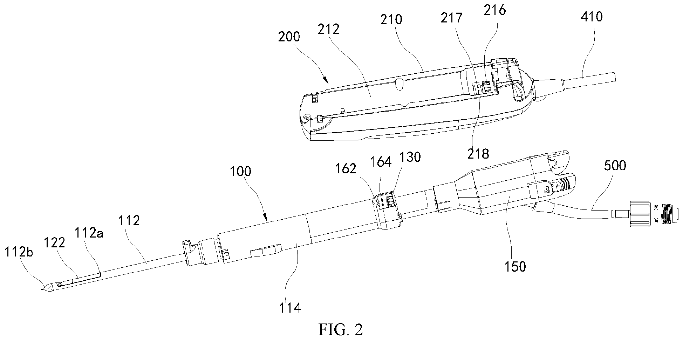

FIG. 1 is a cross-sectional view of an electrosurgical biopsy needle kit according to a first embodiment of the present disclosure, and FIG. 2 is an exploded view of the electrosurgical biopsy needle kit shown in FIG. 1. As shown in FIGS. 1 and 2, the electrosurgical biopsy needle kit according to the first embodiment of the present disclosure includes an electrosurgical biopsy needle 100 and a handle 200. The electrosurgical biopsy needle 100 mainly consists of a puncture assembly 110 and a cutting assembly 120.

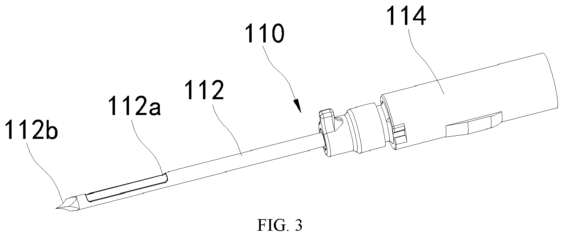

As shown in FIG. 3, the puncture assembly 110 includes a puncture tube 112 and a housing 114. The puncture tube 112 has a front end away from the housing 114 and a rear end adjacent to the housing 114. The front end of the puncture tube 112 is provided with a tip portion 112b. The tip portion 112b punctures a tissue with the assistance of an ultrasound imaging or an X-ray imaging device. A tube wall of the puncture tube 112 adjacent to the front end is provided with a sampling groove 112a. The rear end of the puncture tube 112 extends into and fixed in the housing 114. In this embodiment, the puncture tube 112 is made of conductive material (such as stainless steel), so as to form a second electrode.

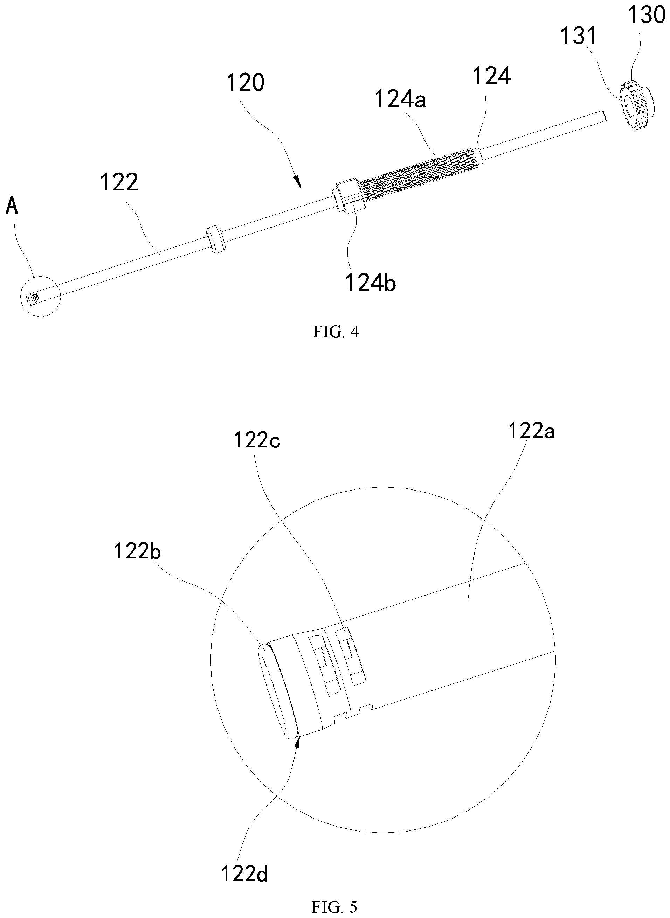

As shown in FIGS. 4 and 5, the cutting assembly 120 includes a cutting member 122. The cutting member 122 is mounted within the puncture tube 112 and can move back and forth along an axis of the puncture tube 112. The cutting member 122 includes an insulated cutting member body 122a. The cutting member body 122a is shaped as a tube, a sheet, or a thin rod. The cutting member 122 further includes a cutting blade 122b provided on a front end opening 122d of the cutting member body 122a. The cutting blade 122b is made of conductive material, so as to form a first electrode.

The electrosurgical biopsy needle 100 further includes a first interface 162 and a second interface 164. The first interface 162 and the second interface 164 are electrically connected to the cutting blade 122b and the puncture tube 112, respectively. Any one of the first interface 162 and the second interface 164 is configured to be directly or indirectly connected to a high-frequency output terminal of a host 300, and the other one is configured to be directly or indirectly connected to a high-frequency input terminal of the host 300. If the first interface 162 is connected to the high-frequency output terminal, and the second interface 164 is connected to the high-frequency input terminal, the cutting blade 122b is formed as an emitter electrode and the puncture tube 112 is formed as a receiver electrode. On the contrary, if the first interface 162 is connected to the high-frequency input terminal, and the second interface 164 is connected to the high-frequency output terminal, the cutting blade 122b is formed as the receiver electrode and the puncture tube 112 is formed as the emitter electrode. The cutting blade 122b, the puncture tube 112, the first interface 162 and the second interface 164 form a circuit, so as to conduct high-frequency energy.

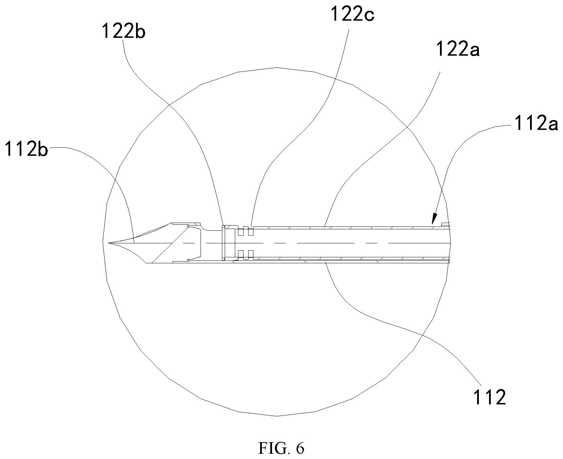

As shown in FIG. 6, when the high-frequency wave is input from the first interface 162 or the second interface 164, a high-frequency electric wave is generated between the cutting blade 122b and an edge of the sampling groove 112a of the puncture tube 112. When the high-frequency electric wave passes through the tissue in the sampling groove 112a, due to the resistance of the tissue to the high-frequency electric wave, the water molecules in the tissue instantly vibrate rapidly, and the water molecules in the cells evaporate, thereby destroying the cells or causing the cells to volatilize. When the high-frequency electric wave reaches the water molecules in the cells, a resistance of the water molecules to the high-frequency electric wave is generated, such that the high-frequency electric wave energy is converted into mechanical energy. As a result, the water molecules instantly vibrate rapidly, and the water molecules in the cells are transformed from liquid water to gaseous water in an instant. The cells are broken up as the water molecules expanding in volume, the tissue separation is formed, thereby realizing the cutting. Furthermore, utilizing the thermal effect, the cells of the localized soft tissues is coagulated and the protein is denaturated, thereby achieving effective hemostasis.

Compared with conventional rotary cutting electrosurgical biopsy needles, the electrosurgical biopsy needle 100 according to the present disclosure can more effectively crush the calcification foci, and can play a traumatic coagulation role due to thermal effect, thus effectively avoiding the risk of intraoperative bleeding and postoperative hematoma. Compared with existing electrosurgical biopsy needles 100, the electrosurgical biopsy needle 100 according to the present disclosure uses the puncture tube 112 as one electrode, and uses the cutting blade 122b of the cutting member 122 as the other electrode. Cutting is performed using the high-frequency electric wave between the cutting blade 122b and the puncture tube 112. An additional metal plate is not required during operation, which can avoid ambustion of the human body due to the poor fitting of the metal plate with human skin. The safety is good and the operation is convenient. Besides, the structure of the product is simple and the cost is low.

Further referring to FIG. 5, the cutting blade 122b in this embodiment is shaped as a circle extending along a periphery of the front end opening 122d. The cutting blade 122b is relatively long and the cutting efficiency is high. Furthermore, when the cutting member 122 moves from back to forward along an axial direction thereof, the tissue cut by the cutting blade 122b is directly squeezed into the front end opening 122d, and is then sucked into a collection box 150 via vacuum. The cutting blade 122b in this embodiment is formed by bending a metal wire (such as stainless steel wire, tungsten wire). The cutting blade 122b is directly fixed on an end surface of the front end opening 122d. The cutting blade 122b with this structure has the advantages of simple structure and good reliability.

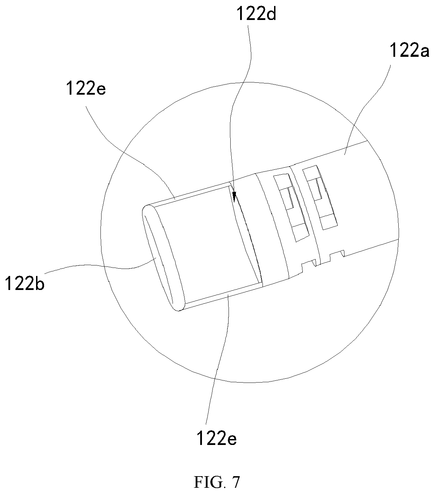

FIG. 7 shows a perspective view of a cutting blade 122b with a second structure. As shown in the figure, the cutting blade 122b is relatively provided on a front side of the front end opening 122d at a certain distance. At least one electrically conductive leg 122e is provided between the cutting blade 122b and the front end opening 122d. One end of the leg 122e is electrically connected to the cutting blade 122b, and the other end thereof is electrically connected to the first interface 162. The leg 122e serves as both a support and a conductor. In this embodiment, two legs 122e are provided, and a line connecting the two legs 122e extends through a center of the cutting blade 122b.

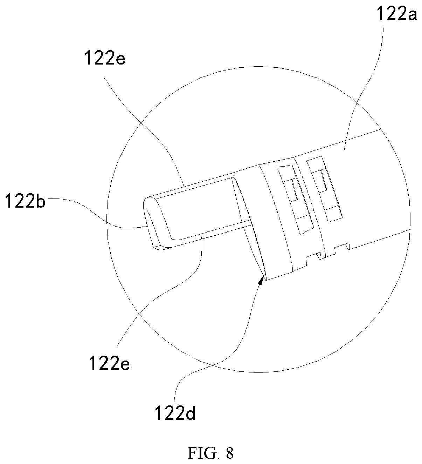

FIG. 8 shows a perspective view of a cutting blade 122b with a third structure. As shown in the figure, the cutting blade 122b is shaped as an arc. Three legs 122e are provided, one end of two of the legs 122e is connected to both ends of the arc, and one end of the remaining one of the legs 122e is connected to an apex of the arc. The cutting blade 122b with this structure is more robust.

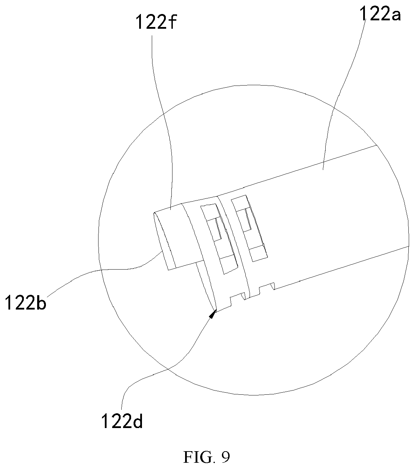

FIG. 9 shows a perspective view of a cutting blade 122b with a fourth structure. As shown in the figure, the front end opening 122d of the cutting member 122 is provided with a closed or non-closed metal sheet 122f extending along the periphery of the front end opening 122d. The cutting blade 122b is formed at a front end of the metal sheet 122f. The metal sheet 122f is electrically connected to the first interface 162. This kind of cutting blade 122b has a great strength and meanwhile further plays a role of mechanical cutting.

In addition to the aforementioned four structures, the cutting blade 122b can also be the following structures: the cutting member body 122a includes a tube body made of metal and an insulation layer wrapping a surface of the tube body. The front end opening 122d of the tube body is exposed and processed into a tip-shape, so as to form the cutting blade 122b. The structure of this cutting member 122 is simpler.

In this embodiment, the cutting member body 122a includes a tube body (not shown in the figure) made of metal (such as stainless steel) and an insulation layer (not shown in the figure) wrapping the surface of the tube body. The tube body is electrically connected to the cutting blade 122b and the first interface 162. Preferably, the cutting blade 122b is welded to the front end of the tube body. As an alternative, the cutting member body 122a is made of an insulation material (ceramic material, hard plastic). The cutting blade 122b is fixed on the end surface of the front end opening 122d. At least one connecting wire (not shown in the figure) is provided in a tube wall of the cutting member body 122a. One end of the connecting wire is electrically connected to the cutting blade 122b, and the other end is electrically connected to the first interface 162. Preferably, the cutting blade 122b and the connecting wire are integrally formed.

In order to facilitate the entry of the cut tissue into the cutting member 122, a circumferential wall of the front end opening 122d of the cutting member body 122a is provided with at least one vent port 122c.

As shown in FIGS. 1 and 2, the first interface 162 includes a first pin, the second interface 164 includes a second pin. The first pin may be electrically connected to the cutting blade 122b through a wire. Alternatively, cutting member body 122a includes a tube body made of metal and an insulation layer wrapping the surface of the tube body, and no insulation coating is coated at a position of the tube body adjacent to the first pin. An inner end of the first pin is in contact with the tube body to implement the electrical connection, and the front end of the tube body is electrically connected to the cutting blade 122b, thereby implementing the electrical connection between the first pin and the cutting blade 122b. An inner end of the second pin is electrically connected to the puncture tube 112 through a wire (not shown in the figures). Both outer ends of the first pin and the second pin are exposed from the housing 114.

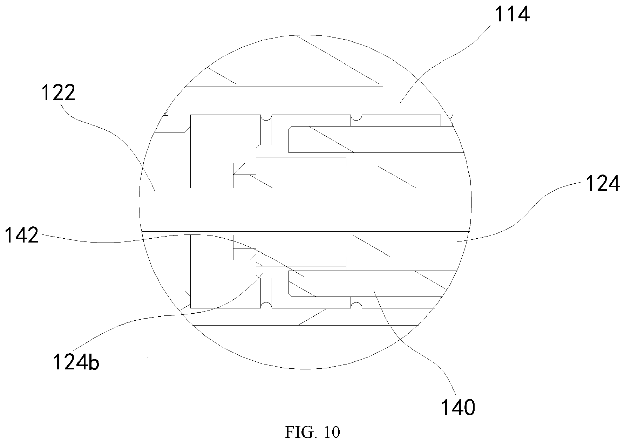

As shown in FIGS. 1, 4 and 10, in this embodiment, the electrosurgical biopsy needle 100 further includes a transmission mechanism configured to convert the rotational torque into linear motion of the cutting member 122. For example, the transmission mechanism includes a pushing rod 124, a first gear 130, and a stop-rotation mechanism. The pushing rod 124 is fixed and sleeved on the cutting member 122 extending into the housing 114. The pushing rod 124 is provided with an external thread 124a. A center of the first gear 130 is provided with a threaded hole 131. An internal thread of the threaded hole 131 is engaged with the external thread 124a on the pushing rod 124. The stop-rotation mechanism is configured to limit the cutting member 122 from rotating relative to the puncture tube 112. When the first gear 130 rotates, the pushing rod 124 moves axially along an axis of the first gear 130, thus transforming the rotatory motion into a linear motion, thereby realizing forward and backward movement of the cutting assembly 120. As shown in the figures, an end of the pushing rod 124 adjacent to the front side of the puncture tube 112 is provided with at least one limit groove 124b extending in the axial direction. A shaft sleeve 140 is fixed in the housing 114. The shaft sleeve 140 is sleeved on the pushing rod 124. An inner wall of the shaft sleeve 140 is provided with a limiting projection 142 extending in the axial direction. The limiting projection 142 is mated with the limit groove 124b to form the stop-rotation mechanism, which limits the cutting member 122 from rotating relative to the puncture tube 112.

The electrosurgical biopsy needle 100 in this embodiment further includes a collection box 150. An inlet of the collection box 150 is connected to the rear end of the cutting member 122, and the cut tissue enters the collection box 150 through the cutting member 122.

A gas channel for gas circulation is formed between an outer wall of the cutting member 122 and an inner wall of the puncture tube 112. The electrosurgical biopsy needle 100 further includes an air valve mechanism for making a rear opening of the gas channel open or closed to the outside atmosphere. The air valve mechanism in this embodiment may be the air valve mechanism disclosed in the Chinese Patent Publication No. CN209285578U.

As shown in FIGS. 1 and 2, the handle 200 includes a housing 210, a driving device, a circuit board (not shown in the figures), a first cable 410, a third interface 217, and a fourth interface 218. A side of the housing 210 is provided with a latching groove 212 configured to accommodate the housing 114 of a biopsy needle. The driving device is accommodated in the housing 210. The driving device includes a motor 214 and a second gear 216. The second gear 216 is mounted on an output shaft of the motor 214. The second gear 216 is partially exposed from the housing 210, and is engaged with the first gear 130 of the electrosurgical biopsy needle 100. The circuit board is connected to the motor 214, the first cable 410, the third interface 217 and the fourth interface 218. In this embodiment, the third interface 217 is a first jack mated with the first pin, and the fourth 218 is a second jack mated with the second pin. When the electrosurgical biopsy needle 100 is mounted in the latching groove 212, the first pin and the second pin are inserted into the first jack and the second jack, respectively.

In one embodiment, the cutting member 122 can also rotate relative to the puncture tube 112.

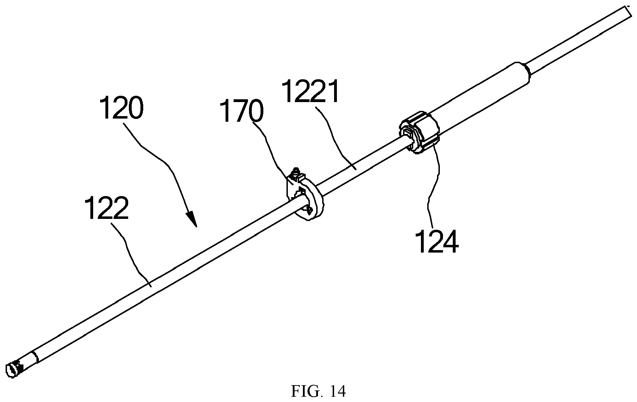

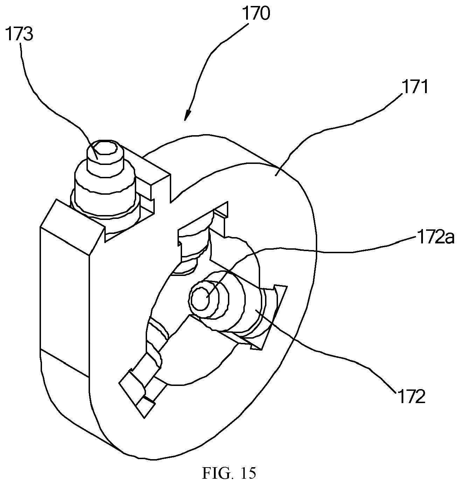

Referring to FIGS. 14 and 15, in one embodiment, the cutting member body 122a is provided with an electrode sleeve ring 170. The first electrode is connected to the first interface 162 through the electrode sleeve ring 170. Any one of the first interface 162 and the second interface 164 is further configured to be directly or indirectly connected to the high-frequency output terminal of the host 300, and the other one is further configured to be directly or indirectly connected to an electro-coagulation input terminal of the host 300. If the first interface 162 is connected to the high-frequency output terminal, and the second interface 164 is connected to the electro-coagulation input terminal, the cutting blade 122b is formed as the emitter electrode and the puncture tube 112 is formed as the receiver electrode. On the contrary, if the first interface 162 is connected to the electro-coagulation input terminal, and the second interface 164 is connected to the high-frequency output terminal, the cutting blade 122b is formed as the receiver electrode and the puncture tube 112 is formed as the emitter electrode. The cutting blade 122b, the puncture tube 112, the first interface 162 and the second interface 164 form the circuit, so as to conduct current.

When it is required to stop bleeding, current is input to the first interface 162 or the second interface 164, and is transmitted from the first interface 162 or the second interface 164 to the first electrode or the second electrode. Due to the presence and electrical conductivity of body fluids and tissues between the first electrode and the second electrode, the first electrode and the second electrode are connected and current is formed in the human body. Furthermore, since an area of the first electrode is smaller than an area of the second electrode, the current adjacent to the first electrode is energy-dense and releases heat in the body, which heats the traumatic tissues and realizes the coagulation of the tissues of the body, thereby achieving the object of hemostasis.

Referring to FIGS. 14 and 15, in one embodiment, the electrode sleeve ring 170 includes an annular mount 171 and at least one elastic contact assembly 172 arranged in a circumferential direction provided on the mount 171. The elastic contact assembly 172 includes an elastic contact 172a provided in a central hole of the mount 171. Specifically, a rear end of the cutting member body 122a has an electrically conductive area 1221. A head of the elastic contact 172a is in slidably fit with an outer wall of the electrically conductive area 1221. Since the electrode sleeve ring 170 employs this structure, when the cutting member 122 moves axially back and forth and rotates, the head of the elastic contact 172a is always in reliable contact with the electrically conductive area 1221, thus the connection reliability is high. The electrode sleeve ring 170 further includes a connector 173 provided on an outer peripheral surface of the mount 171. The connector 173 is electrically connected to the first pin through a wire. The elastic contact 172a is electrically connected to the connector 173.

Referring to FIGS. 1 and 2, in one embodiment, the puncture assembly 110 further includes a tubular housing 114. The rear end of the puncture tube 112 is inserted and fixed in a front end of the housing 114, and the rear end of the cutting member 122 extends out of the puncture tube 112 and extends into the housing 114. The first interface 162 and the second interface 164 are provided on the housing 114.

Referring to FIGS. 1 and 2, in one embodiment, the first interface 162 includes the first pin, the second interface 164 includes the second pin. The first pin is electrically connected to the electrode sleeve ring 170. The inner end of the second pin is electrically connected to the puncture tube 112 through a wire, and both outer ends of the first pin and the second pin are exposed from the housing 114. For example, the cutting member body 122a includes the tube body made of metal and the insulation layer wrapping the surface of the tube body. The insulation layer is exposed from a rear end of the tube body, so as to form the electrically conductive area 1221. The electrically conductive area 1221 extends through the electrode sleeve ring 170, and can move back and forth and rotate relative to an axis of the electrode sleeve ring 170.

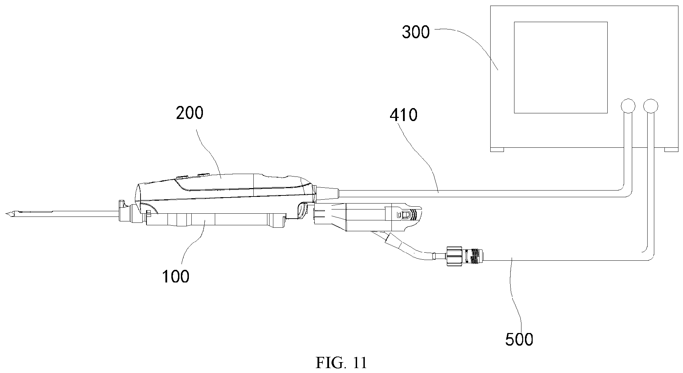

FIG. 11 is a schematic view of a vacuum-assisted breast biopsy system having the electrosurgical biopsy needle kit according to the aforementioned embodiments. As shown in the figure, the vacuum-assisted breast biopsy system according to the present disclosure includes the electrosurgical biopsy needle kit according to the aforementioned embodiments and a host 300. The host 300 is provided with a high-frequency transmitting module (not shown in the figure) and a vacuum generation system (not shown in the figure). A high-frequency output terminal and a high-frequency input terminal of the high-frequency transmitting module are connected to the circuit board in the handle 200 through the first cable 410, and the circuit board is electrically connected to the first jack and the second jack. Therefore, the high-frequency output terminal and the high-frequency input terminal of the high-frequency transmitting module are indirectly connected to the first pin and the second pin. The vent port 122c, the gas channel between the cutting member 122 and the puncture tube 112, and an interior of the cutting member 122 form a gas pathway, which avoids the front end of the cutting member 122 from blocking.

Additionally, the host 300 is further provided with an electro-coagulation module (not shown in the figure) and the vacuum generation system (not shown in the figure). A high-frequency output terminal and an electro-coagulation input terminal of the electro-coagulation module are connected to the circuit board in the handle 200 through a fourth cable (not shown in the figure). Therefore, the high-frequency output terminal and the electro-coagulation input terminal of the electro-coagulation module are indirectly connected to the first pin and the second pin. The vent port 122c, the gas channel between the cutting member 122 and the puncture tube 112, and the interior of the cutting member 122 form a gas pathway, which avoids the front end of the cutting member 122 from blocking.

The working principle of vacuum-assisted breast biopsy system according to the embodiments of the present disclosure is described as follows:

A user firstly punctures the front end of the puncture tube 112 to the position of the operation point under the guidance of an ultrasound equipment. At this time, the front end opening 122d of the cutting member 122 is located at a position most adjacent to the front end of the puncture tube 112, and the sampling groove 112a is in a closed state. A control signal is sent through the control button on the handle 200, and the circuit board controls the motor 214 to start working and the negative pressure is turned on. Through the cooperation of the second gear 216 and the first gear 130, the cutting member 122 of the electrosurgical biopsy needle 100 is driven to move backward, and the sampling groove 112a is opened to a desired size. When the cutting member 122 moves backward, the air valve closes, and the cutting member 122, the collection box 150, the vacuum tube 500, and the vacuum connector of the electrosurgical biopsy needle 100 are sequentially connected, so as to form a vacuum channel, and the tissue specimen is sucked into the sampling groove 112a.

A control signal is sent to start sampling through the handle 200 button, or the software automatically controls the start of sampling. Through the cooperation of the second gear 216 and the first gear 130, the cutting member 122 of the electrosurgical biopsy needle 100 is driven to move forward, such that the cutting blade 122b moves forward. At the same time, the host 300 outputs high-frequency electrical wave, which is transmitted to the cutting blade 122b through the first cable 410, the circuit board, the first jack, and the first pin. The high-frequency electrical wave is generated between the cutting blade 122b and the puncture tube 112, and the lesion tissue is partially or completely removed through the high-frequency electric wave. When the cutting member 122 moves to the position most adjacent to the front end of the puncture tube 112, the air valve mechanism opens. The outside atmosphere enters the front end opening 122d of the cutting member 122 through the vent port 122c and the gas channel between the cutting member 122 and the puncture tube 112, and the interior of the cutting member 122 is vacuum. Therefore, the cut tissue specimen enters the cutting member 122 through the vent port 122c under the pressure difference between the interior and exterior, and is then transported to the collection box 150 for film preparation and pathological analysis and diagnosis. On the one hand, the lesion tissue specimen can be minimally invasively taken out and accurate pathological diagnosis can be made. On the other hand, the lesion can be minimally invasively surgically removed.

Additionally, when it is required to stop bleeding, current is input to the first electrode through the cable, the circuit board, the first jack and the first pin by the electro-coagulation module. Due to the presence and electrical conductivity of body fluids and tissues between the first electrode and the second electrode, the first electrode and the second electrode are connected and an electro-coagulation circuit is formed. Furthermore, since the area of the first electrode is smaller than the area of the second electrode, the current adjacent to the first electrode is energy-dense and releases heat in the body, which heats the traumatic tissues and realizes the coagulation of the tissues of the body, thereby achieving the object of hemostasis.

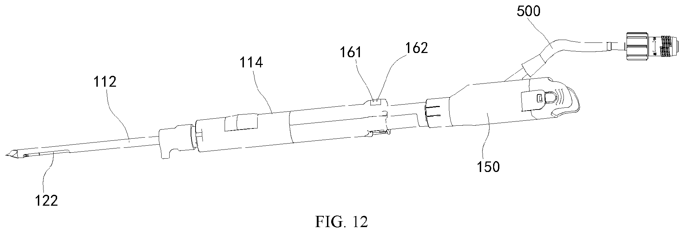

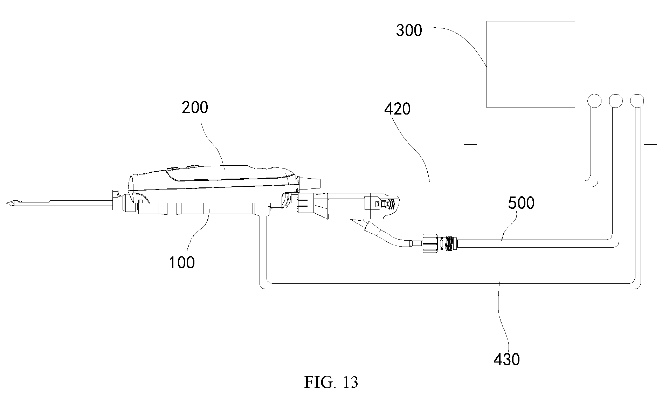

FIG. 12 is a perspective view of an electrosurgical biopsy needle kit according to a second embodiment of the present disclosure, and FIG. 13 is a schematic view of a vacuum-assisted breast biopsy system having the electrosurgical biopsy needle kit shown in FIG. 12. As shown in the figure, the electrosurgical biopsy needle kit in this embodiment is substantially the same as the electrosurgical biopsy needle kit in the embodiments. The difference lies in that the high-frequency circuit of the electrosurgical biopsy needle 100 is directly connected to the host 300. In other words, the first pin and the second pin of the puncture assembly 110 are provided on a side back to the handle 200, the host 300 is connected to the circuit board of the handle 200 through a second cable 420, and is electrically connected to the first pin and the second pin through a third cable 430.

Besides, optionally, the first interface 162 and the second interface 164 of the biopsy needle 100 are directly connected to the host 300. In other words, the first pin and the second pin of the puncture assembly 110 are provided on the side away from the handle 200, the host 300 is connected to the circuit board of the handle 200 through a fifth cable (not shown in the figure), and the high-frequency output terminal and the electro-coagulation input terminal of the electro-coagulation module are electrically connected to the first pin and the second pin through a sixth cable (not shown in the figure).

The above embodiments merely illustrate several embodiments of this disclosure, and the description thereof is specific and detailed, but it shall not be constructed as a limitation on the scope of the disclosure. It should be noted that a plurality of variations and modifications may be made by those skilled in the art without departing from the scope of this disclosure, which are all within the scope of protection of this disclosure.

## Claims

1. An electrosurgical biopsy needle, comprising:
a puncture assembly comprising a puncture tube having a front end and a rear end, a tube wall of the puncture tube is provided with a sampling groove; and
a cutting assembly comprising a cutting member;
wherein the cutting member is capable of moving back and forth along an axis of the puncture tube;
wherein the cutting assembly has a first electrode, and the puncture assembly has a second electrode, the cutting assembly further comprises a first interface and a second interface electrically connected to the first electrode and the second electrode, respectively, any one of the first interface and the second interface is configured to be directly or indirectly connected to a high-frequency output terminal of a host, and the other one is configured to be directly or indirectly connected to a high-frequency input terminal of the host.

2. The electrosurgical biopsy needle according to claim 1, wherein the cutting member comprises a cutting member body and a cutting blade provided on a front end opening of the cutting member body, the cutting member body is insulated, the cutting blade is electrically conductive and forms the first electrode, and the puncture tube is electrically conductive and forms the second electrode.

3. The electrosurgical biopsy needle according to claim 2, wherein the cutting blade is shaped as a circle or an arc extending along a periphery of the front end opening.

4. The electrosurgical biopsy needle according to claim 3, wherein the cutting blade is formed by bending a metal wire.

5. The electrosurgical biopsy needle according to claim 3, wherein the front end opening is provided with a closed or non-closed metal sheet extending along the periphery of the front end opening, the cutting blade is formed at a front end of the metal sheet, and the metal sheet is electrically connected to the first interface.

6. The electrosurgical biopsy needle according to claim 3, wherein the cutting blade is directly fixed on an end surface of the front end opening.

7. The electrosurgical biopsy needle according to claim 3, wherein the cutting blade is relatively provided on a front side of the front end opening at a certain distance, at least one electrically conductive leg is provided between the cutting blade and the front end opening, one end of the leg is electrically connected to the cutting blade, and the other end thereof is electrically connected to the first interface.

8. The electrosurgical biopsy needle according to claim 7, wherein the cutting blade is shaped as a circle, two legs are provided, and a line connecting the two legs extends through a center of the circle.

9. The electrosurgical biopsy needle according to claim 7, wherein the cutting blade is shaped as an arc, three legs are provided, one end of two of the legs is connected to both ends of the arc, and one end of the remaining one of the legs is connected to an apex of the arc.

10. The electrosurgical biopsy needle according to any one of claims 2 to 9, wherein the cutting member body comprises a tube body made of metal and an insulation layer wrapping a surface of the tube body, and the tube body is electrically connected to the cutting blade and the first interface.

11. The electrosurgical biopsy needle according to any one of claims 2 to 9, wherein the cutting member body is made of an insulation material, at least one connecting wire is provided in a tube wall of the cutting member body, one end of the connecting wire is electrically connected to the cutting blade, and the other end thereof is electrically connected to the first interface.

12. The electrosurgical biopsy needle according to any one of claims 1 to 9, wherein the puncture assembly further comprises a tubular housing, the rear end of the puncture tube is inserted and fixed in a front end of the housing, a rear end of the cutting member extends out of the puncture tube and extends into the housing, a pushing rod is fixed and sleeved on the cutting member extending into the housing, a first gear is threadedly mated to the pushing rod, and further comprises a stop-rotation mechanism configured to limit the cutting member from rotating relative to the puncture tube.

13. The electrosurgical biopsy needle according to any one of claims 1 to 9, wherein the cutting member comprises the cutting member body, an electrode sleeve ring is provided on the cutting member body, the first electrode is connected to the first interface through the electrode sleeve ring, any one of the first interface and the second interface is further configured to be directly or indirectly connected to a high-frequency output terminal of a host, and the other one is further configured to be directly or indirectly connected to a high-frequency input terminal of the host.

14. The electrosurgical biopsy needle according to claim 13, wherein a rear end of the cutting member body has an electrically conductive area electrically connected to the first electrode, the electrically conductive area extends through a central hole of the electrode sleeve ring and is capable of moving back and forth and rotating relative to an axis of the electrode sleeve ring, the electrode sleeve ring is provided with at least one elastic contact assembly, the elastic contact assembly comprises an elastic contact provided in the central hole of the electrode sleeve ring, a head of the elastic contact is in slidably fit with an outer wall of the electrically conductive area, and the elastic contact is directly or indirectly connected to the first interface.

15. The electrosurgical biopsy needle according to claim 14, wherein the electrode sleeve ring further comprises a connector provided on an outer peripheral surface of the electrode sleeve ring, the connector is connected to the first interface, and the elastic contact is electrically connected to the connector.

16. The electrosurgical biopsy needle according to any one of claims 1 to 9, wherein the cutting member is capable of rotating relative to the puncture tube.

17. The electrosurgical biopsy needle according to any one of claims 1 to 9, wherein the puncture assembly further comprises a tubular housing, the rear end of the puncture tube is inserted and fixed in a front end of the housing, a rear end of the cutting member extends out of the puncture tube and extends into the housing, and the first interface and the second interface are provided on the housing.

18. The electrosurgical biopsy needle according to claim 17, wherein the first interface comprises a first pin, the second interface comprises a second pin, the first pin is electrically connected to the first electrode, an inner end of the second pin is electrically connected to the puncture tube through a wire, and both outer ends of the first pin and the second pin are exposed from the housing.

19. An electrosurgical biopsy needle kit, comprising a handle, the handle comprising a housing and a circuit board provided in the housing, wherein the electrosurgical biopsy needle kit further comprises the electrosurgical biopsy needle according to any one of claims 1 to 18, the handle further comprises a third interface and a fourth interface mated with the first interface and the second interface, respectively, the third interface and the fourth interface are electrically connected to the circuit board, the electrosurgical biopsy needle is connected to the housing, and the first interface and the second interface are electrically connected to the third interface and the fourth interface, respectively.

20. An electrosurgical biopsy needle kit, comprising a handle, the handle comprising a housing, wherein the electrosurgical biopsy needle kit further comprises the electrosurgical biopsy needle according to any one of claims 1 to 18, and the electrosurgical biopsy needle kit is electrically connected to the housing.

21. A vacuum-assisted breast biopsy system, comprising:
the electrosurgical biopsy needle kit according to claim 19 or 20; and
a host comprising a high-frequency transmitting module configured to generate high frequency, the high-frequency transmitting module has a high-frequency output terminal and the high-frequency input terminal, the high-frequency output terminal and the high-frequency input terminal are connected to the circuit board through a cable and thus indirectly connected to the first interface and the second interface, or the high-frequency output terminal and the high-frequency input terminal are directly connected to the first interface and the second interface through the cable.
